# EUROPEAN PATENT APPLICATION

(11) **EP 4 324 917 A1**
(43) Date of publication of application: **21.02.2024**
(21) Application number: 22788071.3
(22) Date of filing: 31.03.2022
(51) Int. Cl.: C12N 5/10, A61K 35/17, A61P 35/00, C07K 16/10, C07K 16/18, C07K 16/28, C07K 16/46, C07K 19/00, C12N 15/09, C12N 15/13, C12N 15/62, C12N 15/85, C12N 15/86, C12P 21/08

(54) **CELL BANK COMPOSED OF IPS CELLS FOR INTRODUCING T CELL RECEPTOR GENE**

(30) Priority: 16.04.2021 JP 2021069972
(71) Applicant: Thyas Co. Ltd., Kyoto-shi, Kyoto 606-8304 (JP)
(72) Inventor: GONOTSUBO, Ryosuke, Kyoto-shi, Kyoto 606-8304 (JP); OSAWA, Mitsujiro, Kyoto-shi, Kyoto 606-8304 (JP); HITOSHI, Yasumichi, Kyoto-shi, Kyoto 606-8304 (JP); KANEKO, Shin, Kyoto-shi, Kyoto 606-8304 (JP)
(74) Representative: Kraus & Lederer PartGmbB
(86) International application number: PCT/JP2022/016392
(87) International publication number: WO 2022/220146

(57) **Abstract**

[Problem]

To provide a cell bank for rapid production of regenerative T cells into which an antigen-specific T cell receptor of an individual patient have been introduced, said cell bank containing hematopoietic stem cells differentiated and induced from iPS cells, immature T cells and/or mature T cells as intermediates in the production.

[Solution]

A cell bank, which contains one or more kinds of cells selected from the group consisting of iPS cells, hematopoietic stem cells differentiated from iPS cells, immature T cells, and mature T cells is constructed. The iPS cells are obtained by reprogramming peripheral blood mononuclear cells from which B cells and T cells have been removed, or by reprogramming T cells. The transfection of T cell receptor gene into the hematopoietic stem cells or the immature T cells uses a viral vector, a transposon vector, or genome editing technique. The transfection of T cell receptor gene into the mature T cells uses genome editing technique.

## Description

### TECHNICAL FIELD

The present invention relates to a cell bank comprising hematopoietic stem cells derived from iPS cells, immature T cells and mature T cells as intermediates for producing T cells (regenerated T cells) from induced pluripotent stem cells (iPS cells) into which T cell receptor (TCR) genes that recognize antigens derived from tumors or pathogens are transfected. Furthermore, the present invention relates to the use of the cell bank comprising hematopoietic stem cells, immature T cells and mature T cells for production of regenerated T cell formulations for use in prevention and/or treatment of cancer.

### BACKGROUND OF THE INVENTION

T cells play a central role in immune response to foreign pathogens such as bacteria or viruses or abnormal cells such as cancer cells. Therefore, decrease in the function of T cells is considered to contribute to the onset of cancer and pathogen infection. T cell replenishment therapy or regenerative therapy to a patient with a disease caused by decrease in the function of T cells can be an extremely effective means for amelioration of conditions and treatment of the disease in the patient.

In research using humans and mice, it is known that in the case of performing T cell replenishment therapy for cancer or infectious diseases, high therapeutic effects can be achieved by using the T cells that specifically recognize antigens possessed by cancer cells or abnormal cells infected with foreign pathogens such as bacteria or viruses. On the other hand, the difficulty in securing enough amount of T cells, the long time required to produce T cells, and exhaustion of T cells such as decrease in proliferation ability in T cells and decrease in immune response to antigens such as target cells when cells derived from patients are used as materials have become obstacles in T cell replenishment therapy.

To overcome the above obstacles in T cell replenishment therapy, T cell replenishment therapy using regenerated T cells differentiated into T cells after establishing iPS cells from antigen-specific T cells and proliferating the iPS cells, or T cell replenishment therapy using regenerated T cells differentiated into T cells from iPS cells into which TCR for recognizing target antigens has been transfected have been proposed. By using T cells specific to target antigens or TCRs that recognize target antigens as raw materials for producing iPS cells, it is possible to produce regenerated T cells which exhibit the same antigen specificity as the original T cells (patent document 1 and non-patent document 1).

When producing regenerated T cells from iPS cells, long-term differentiation cultures are required (patent document 2). The long production period of regenerated T-cells is not particularly problematic because if an established TCR is transfected into allogeneic iPS cells, the produced regenerated T cells can be preserved. On the other hand, if an established TCR is used for a patient different from an individual from which the TCR is derived, there is a risk of side effects by alloreactions. Thus, for avoiding the risk, attentions have been focused on methods which use antigen-specific TCRs for individual patient. Each individual has different gene mutations in cancer, which results in neoantigen, the target of T cell therapy. Administrations of vaccine against neoantigen have demonstrated high drug efficacy so far. Therapeutic agents produced by methods which use antigen-specific TCRs for individual patient have great benefits in cancer treatment.

### PRIOR ART LITERATURES

### Patent Documents

1. WO 2011/096482 (a pamphlet)
2. WO 2013/176197 (a pamphlet)

### Non-Patent Documents

1. Nishimura T, et al. Generation of rejuvenated antigen-specific T cells by reprogramming to pluripotency and redifferentiation. Cell Stem Cell. 2013; 12:114-126.

### SUMMARY OF THE INVENTION

### Problems to be Solved by the Invention

In the treatment of cancer and infectious diseases using regenerated T cells (iPS-T cells) produced via iPS cells, it is important to use the TCRs of each patient which specifically recognize target antigens in tumor cells or tissue, as well as infected cells and sites in order to ensure safety and effectiveness of regenerative T cell replenishment therapy in treatment. In addition, when an onset or a recurrence of cancer is detected, it is important to promptly initiate iPS-T cell replenishment therapy in order to improve treatment outcomes. However, the production of cell formulations using antigen-specific TCRs for individual patient needs to be carried out for each patient, which is labor and time requiring. These problems have hampered the development of cell formulations. Particularly in allogeneic regenerated T cell preparations, the long production period has been a problem.

The present invention is directed to the construction of a cell bank that can provide a cell group comprising hematopoietic stem cells differentiated from iPS cells, immature T cells and mature T cells, as production intermediates for the rapid production of iPS-T cells into which antigen-specific TCRs for individual patient have been transfected. Furthermore, the present invention includes use of the cell bank for production of regenerated T cell formulations from said production intermediates for use in prevention and/or treatment of cancer or infectious diseases.

Recent studies have shown that for one antigen epitope, multiple T cell clones (5 to 10 clones) recognize an antigen, respectively with different TCR. It has also been shown that for one antigen epitope, each patient has a different TCR repertoire. In addition, cancer cells have mutually different gene mutations in individual patients, resulting in neoantigen, which is a preferable target of T cell therapy. It is highly likely that regenerated T cells produced using TCR obtained from individual patients are the T cells with an optimal TCR to injure a target of T cell therapy in the body of a corresponding patient. It is evident that regenerated T cells produced using TCR obtained from individual patients do not cause an alloreaction which attacks recipient cells. Thus, the benefit of producing regenerated T cells using TCRs obtained from individual patients is extremely high with respect to safety and drug efficacy in prevention and/or treatment of cancer or infectious diseases.

In the present circumstances, a long period of time is required for producing regenerated T cells using iPS cell clones derived from cells obtained from patients. In order to promptly provide regenerated T cell replenishment therapy to patients in need thereof, shortening the production period has become a major challenge.

### Means for Solving the Problems

The present inventors have made the following findings and completed the present invention. Regenerated T cells can be produced by obtaining different TCRs with antigen specificity from T cell populations reactive with individual patient's tumor-associated or pathogen-specific antigens, differentiating and inducing the TCR from iPS cells, and transfecting into prestored hematopoietic stem cells, immature T cells or mature T cells, or replacing with endogenous TCRs. The production period of regenerated T cells can be greatly shortened by constructing a cell bank from hematopoietic stem cells, immature T cells, and mature T cells which are previously differentiated and induced from iPS cells. Homogenization and standardization of regenerated T cell formulations are facilitated by using the cells contained in a cell bank as intermediates for producing regenerated T cell formulations. The present invention can also be applied to the production of regenerated T cells into which established TCRs have been transfected and regenerated T cells for transfecting chimeric antigen receptors (CARs).

That is, the objects of the present invention are achieved by following inventions.
[1] A cell bank composed of cells for transfecting a T cell receptor gene, wherein the cells are one or more kinds of cells selected from the group consisting of iPS cells, hematopoietic stem cells differentiated from the iPS cells, immature T cells, and mature T cells.
[2] The cell bank according to [1], wherein the cells are those for further transfecting a chimeric antigen receptor gene.
[3] The cell bank according to [1] or [2], wherein the iPS cells are peripheral blood mononuclear cells of a subject and are those obtained by reprogramming the peripheral blood mononuclear cells from which B cells and T cells have been removed.
[4] The cell bank according to [3], wherein the transfection of T cell receptor gene into the iPS cell clone or the hematopoietic stem cells differentiated from the iPS cell clone or immature T cells uses a viral vector, a transposon vector, or genome editing technique.
[5] The cell bank according to [1] or [2], wherein the iPS cells are those obtained by reprogramming T cells of a subject.
[6] The cell bank according to [5], wherein the transfection of T cell receptor gene into the mature T cells uses genome editing technique.
[7] The cell bank according to any one of [1] to [6], wherein the iPS cells are iPS cell clones with good differentiation efficiency into mature T cells.
[8] The cell bank according to any one of [1] to [7], wherein the cells are cryopreserved.
[9] The cell bank according to [5], wherein the cells are those genetically modified so that expression of endogenous T cell receptors can be controlled.
[10] The cell bank according to any one of [1] to [9], wherein the hematopoietic stem cells and the immature T cells are the cells which do not express a T cell receptor.
[11] The cell bank according to [5], wherein the mature T cells express a T cell receptor which does not recognize non-tumor cells derived from a subject different from a subject from which the mature T cells are derived.
[12] The cell bank according to [11], wherein the mature T cells recognize a single antigen.
[13] The cell bank according to [12], wherein the single antigen is influenza virus antigen, EB virus antigen, HPV antigen, HBV antigen, HCV antigen, HIV antigen, coronavirus antigen, or HTLV antigen.
[14] The cell bank according to any one of [1] to [13], wherein the hematopoietic stem cells are CD34/CD43 double-positive.
[15] The cell bank according to any one of [1] to [14], wherein the immature T cells are CD8 α chain/β chain double-positive.
[16] The cell bank according to any one of [1] to [15], wherein the mature T cells are CD8 α chain/β chain double-positive and TCR α chain/β chain double-positive.
[17] The cell bank according to any one of [1] to [16], wherein the T cell receptor gene is a T cell obtained from a subject and prepared for each single cell from a T cell population having reactivity to a tumor-related antigen.
[18] The cell bank according to any one of [1] to [16], wherein the T cell receptor gene is prepared for each single cell from a T cell population having reactivity to the tumor-related antigen by contacting T cells obtained from a subject with a tumor-related antigen.
[19] The cell bank according to any one of [1] to [16], wherein the T cell receptor gene is prepared for each single cell from a T cell population having reactivity to the tumor-related antigen by contacting the T cell obtained from a subject to which a tumor-related antigen was administered with the tumor-related antigen.
[20] The cell bank according to any one of [17] to [19], wherein the tumor-related antigen is selected from the group consisting of GPC3, WT1, XAGE1, LMP2, NY-ESO-1, EB virus antigen and neoantigen, as well as peptide fragments thereof.
[21] The cell bank according to any one of [17] to [19], wherein the tumor-related antigen is EYILSLEEL (SEQ ID NO: 1) which is an HLA-A24-binding GPC3 peptide, FVGEFFTDV (SEQ ID NO: 2) which is an HLA-A2-binding GPC3 peptide, or a mixture thereof.
[22] The cell bank according to any one of [17] to [19], wherein the T cell population is CD3/CD137 double-positive.
[23] The cell bank according to any one of [17] to [19], wherein the T cell population binds to MHC tetramer or MHC Dextran^{®} which forms a complex with the tumor-related antigen peptide.
[24] The cell bank according to any one of [1] to [23], wherein the subject providing cells for obtaining the iPS cells and the subject providing cells for preparing the T cell receptor gene are the same individual.
[25] The cell bank according to any one of [1] to [23], wherein the subject providing cells for obtaining the iPS cells and the subject providing cells for preparing the T cell receptor gene are separate individuals from each other.
[26] The cell bank according to any one of [1] to [25], wherein the cells for transfecting a T cell receptor gene or a T cell receptor gene and a chimeric antigen receptor are intermediates for producing T cell formulations used in prevention and/or treatment of cancer.
[27] Use of the cell bank according to any one of [1] to [26] for production of T cell formulations used in prevention and/or treatment of cancer.
[28] Regenerated T cells produced from the cell bank according to any one of [1] to [26].
[29] A pharmaceutical composition containing the regenerated T cells according to [28].
[30] A method for preventing or treating cancer using the pharmaceutical composition according to [29].

### EFFECTS OF THE INVENTION

Since the cell bank of the present invention contains the hematopoietic stem cells, immature T cells and/or mature T cells which are differentiated and induced from iPS cells and pre-stored, TCR collected from subjects to be treated for cancer therapy or infectious disease or established TCR or CAR can be transfected into these cells to produce regenerated T cells in a short time. Accordingly, the time required from the production of regenerated T cells into which antigen-specific TCR has been transfected, to use of the regenerated T cells in treatment can be dramatically shortened and the amount of regenerated T cells required for the treatment can be stably ensured. In addition, the cell bank of the present invention can be constructed using iPS cell clones induced either from non-T non-B cells or monocytes or from T cells. If TCR is transfected to hematopoietic stem cells differentiated from iPS cell clones induced from non-T non-B cells or monocytes, immature T cells and mature T cells, in case of expanded culture of these cells after being differentiated into regenerative T cells, reconstitution of the TCR gene is less likely to occur. Accordingly, the antigen specificity of the transfected TCR is maintained. As a result, the safety of regenerated T cell formulations is increased. In addition, by using the cells that constitute the cell bank of the present invention, regenerated T cells with less exhaustion caused by expanded culture can be produced. Furthermore, when producing regenerated T cells using iPS cells that constitute the cell bank of the present invention, since iPS cell clones with good differentiation efficiency into T cells are selected in advance and used as the cells that constitute a cell bank, it becomes possible to minimize the influence of variations in cell yield and degree of cell differentiation between production batches of regenerated T cells, and individual differences in subjects from whom cells are collected on the quality etc. of the resulting regenerated T cells. Additionally, by using the cell bank of the present invention, it becomes possible to produce efficiently and quickly the T cells having the TCRs that recognize antigens and kill targets efficiently.

The transfection of the cDNA encoding TCR into cells is carried out in differentiated cells derived from the iPS cells which are differentiated and induced previously from iPS cells and constitute the cell bank of the present invention, i.e., the hematopoietic stem differentiated from iPS cells, immature T cells and mature T cells. Since the cells that constitute the cell bank are differentiated and induced from cloned iPS cells, the quality of the cells is more homogeneous and stable. For differentiated cells derived from the iPS cells, i.e., the hematopoietic stem differentiated from iPS cells, immature T cells and mature T cells, the number of cells used to transfect TCR in one time is 10⁷ to 10⁹ or more. The cell bank of the present invention can comprise the number of cells required for 1000 or more times of TCR transfection in a dispensed form. Therefore, reduced cost for performing TCR transfection in one time can be estimated. Therefore, it becomes possible to prepare quickly and easily the regenerated T cells specific to an antigen that is expressed even for tumor antigenic change or development of therapy resistance or cancer recurrence in cancer patients being treated in the cancer treatment to which the regenerated T cell replenishment therapy is applied.

With respect to the TCR transfected to the cells contained in the cell bank of the present invention, the subject from whom the T cells used to prepare the cDNA encoding the TCR is collected may be the same individual as the subject who is the cancer patient to be treated by the regenerated T cell replenishment therapy and they may be separate individuals. Thus, a safe and effective TCR with optimal antigen specificity for individual cancer patients can be selected.

### BRIEF DESCRIPTION OF THE DRAWINGS

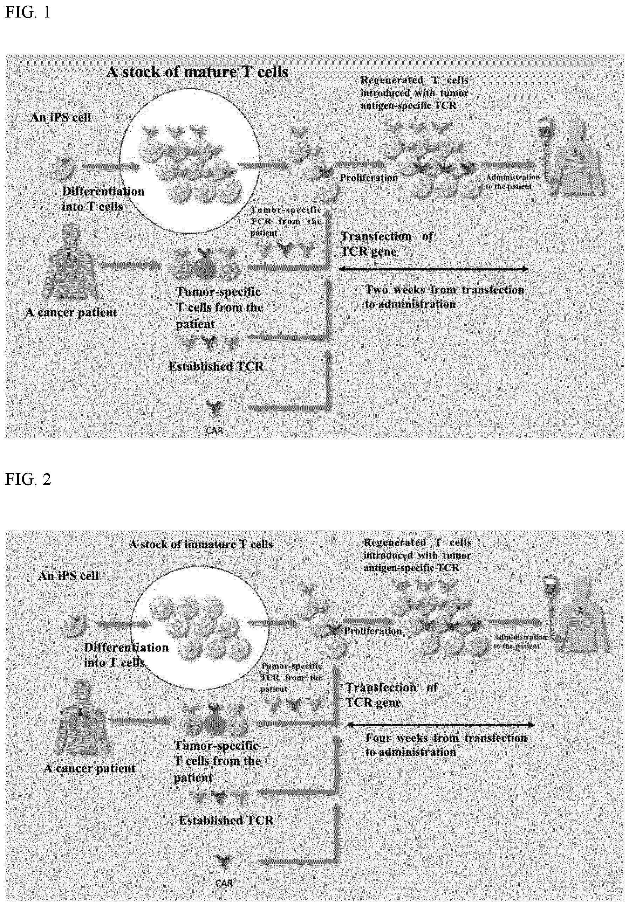

FIG. 1 is a diagram of producing tumor-specific regenerated T cells from mature T cells that are differentiated and induced from iPS cells and constitute a cell bank. Transfection of tumor-specific TCR into mature T cells is carried out by gene replacement using genomic modification techniques, such as CRISPR/Cas9. The time required from TCR transfection to cells to administration of cells induced with TCR to patients is about two weeks. The TCRs expressed in mature T cells are preferably those that do not induce any alloreaction. The TCRs transfected into cells are those obtained from a cancer patient with whom administration of a regenerated T-cell formulation is scheduled, established cancer-specific ones, or cancer-specific CARs.
FIG. 2 is a diagram of producing tumor-specific regenerated T cells from immature T cells that are differentiated and induced from iPS cells and constitute a cell bank. Transfection of tumor-specific TCR into immature T cells is carried out by gene replacement using genomic modification techniques, such as CRISPR/Cas9 or lentiviral or transposon vectors. The time required from TCR transfection to cells to administration of cells induced with TCR to patients is about four weeks. The TCRs transfected into cells are those obtained from a cancer patient with whom administration of a regenerated T-cell formulation is scheduled, established cancer-specific ones, or cancer-specific CARs.
FIG. 3 shows a step of transfecting a TCR gene into hematopoietic stem cells, immature T cells or mature T cells using a transposon.
FIG. 4 shows a phenotype of mature T cells induced from iPS cells (CD8 positive cytotoxic T cells). The mature T cells that constitute a cell bank take the phenotype shown in FIG. 4.
FIG. 5 shows the results of analyzing telomere as a cell aging marker in mature T cells induced from iPS cells.
FIG. 6 shows the results of analyzing the expressions of PD-1 and TIGIT molecules as cell exhaustion markers in mature T cells induced from iPS cells.
FIG. 7 shows a phenotype of immature T cells induced from iPS cells (CD8 positive immature T cells). The immature T cells that constitute a cell bank take the phenotype shown in FIG. 7.
FIG. 8 shows a phenotype of mature T cells derived from iPS cells in which a GPC3 antigen-specific TCR gene has been transfected at stage of mature T cells. The CD19 gene is a gene incorporated in tandem with the TCR gene into the same picggyBac^{®} transposon vector and is used as a marker for gene insertion into a host chromosome and gene expression.
FIG. 9 illustrates a method for selecting the iPS cell clones that demonstrate high differentiation efficiency into T cells in the step of producing regenerated T cells from iPS cells in which peripheral blood T cells have been reprogramed.
FIG. 10 illustrates a method for performing genome editing on iPS cell clones selected as the cells with high differentiation efficiency into T cells and producing regenerated T cells in the step of producing regenerated T cells from iPS cells in which peripheral blood T cells have been reprogramed. The regenerated T cells are taken as a master cell bank of host T cells.
FIG. 11 illustrates a method for producing regenerated T cells which recognize cancer antigens from host T cells.
FIG. 12 shows an outline of a step of producing regenerated T cells from allogeneic iPS cells in which peripheral blood T cells have been reprogramed. The differentiated T cells are taken as a master cell bank of host T cells.

### EMBODIMENTS FOR CARRYING OUT THE INVENTION

In the present invention, a "cell bank" is a cell assembly of multiple types composed of cells for transfecting T cell receptor genes. CARs can also be transfected into the cells that constitute a cell bank. The cell bank of the present invention comprises one or more kinds of cells selected from the group consisting of iPS cells, hematopoietic stem cells differentiated from the iPS cells, immature T cells, and mature T cells. Cells contained in the cell bank of the present invention can be used to produce regenerated T cells for use in prevention and/or treatment of cancer or infectious diseases. The "cell assembly" is something where cells have been divided based on their origin and differentiation stages, etc., dispensed respectively into an independent cell container and integrated. A cell bank of the present invention can be stored in any facility or storage where cells can be cryopreserved.

### Cells

In the present invention, the "mature T cells" that constitute a cell bank are the cells which are differentiated and induced from iPS cells, and are the cells which express a functional TCR on the cell surface that express CD3 and CD4 or CD8 as a cell surface antigen and can induce cell proliferation, cytokine production and cytotoxicity in response to an antigen presented on a major histocompatibility complex. TCR includes heterodimers consisting of α and β chains and heterodimers consisting of γ and δ chains. T cells with the TCR consisting of α and β chains are referred to as αβ type T cells and T cells with TCR consisting of γ and δ chains are referred to as γδ type T cells. In one aspect of the present invention, the mature T cells of the present invention are preferably CD3/αβ type T cells, but may also be CD3/yδ type T cells. Mature T cells may be the cells expressing an endogenous TCR wherein TCR gene reconstruction has ended, and may also be the cells expressing a TCR which is transfected exogenously.

In the present invention, the "immature T cells" that constitute a cell bank are the cells which are differentiated and induced from iPS cells. However, they are on route to differentiation into T cells and do not express TCR on cell surface. Immature T cells include T cells at each stage from CD4/CD8 double negative cells and CD4/CD8 double-positive cells to CD8 single positive cells. CD8 expressed in immature T cells are preferably CD8αβ heterodimers, but may also be CD8αα homodimers.

In the present invention, the "hematopoietic stem cells" that constitute a cell bank are the cells which can differentiate into hematopoietic cells, such as lymphocytes, eosinophils, neutrophils, basophils, erythrocyte, and megakaryocytes. Hematopoietic stem cells and hematopoietic progenitor cells (HPC) are not distinguished. They are referred to the same cells unless otherwise indicated. Hematopoietic stem cells or hematopoietic progenitor cells are recognized, for example, by being double-positive of CD34 and CD43, which are surface antigens.

In the present invention, the "cells which are genetically modified so that expression of endogenous TCRs can be controlled" are genetically modified cells which can suppress or control the expression of endogenous TCRs by a method using microRNA etc. or by replacing a promoter of endogenous TCR with a tetracycline-controlled promoter etc.

In the present invention, the TCRs expressed by the mature T cells that constitute a cell bank recognize a single antigen and do not cause or hardly cause an alloreaction. Antigens recognized by the TCR include influenza virus antigens, EB virus antigens, HPV antigens, HBV antigens, HCV antigens, HIV antigens, coronavirus antigens, HTLV antigens, and cancer antigens.

In the present invention, an antigen recognized by a TCR transfected into cells that constitute a cell bank is an antigen that is expressed in a tumor-specific or non-specific manner, such as an antigen derived from a protein overexpressed in tumor cells and a mutant thereof, an antigen derived from a tumor virus, a certain type of differentiated antigen, or a new tumor-related antigen (neoantigens) due to gene mutation and splice abnormality, etc. Tumor-related antigens may also be described herein as tumor antigens. In the case of a protein antigen, this may be a fragmented peptide (a peptide fragment). Examples of the antigens that are expressed in a tumor-specific or non-specific manner include, but are not limited thereto, WT1, GPC3, XAGE1, MUC1, MUC5AC, MUC6, EGFRvIII, HER-2/neu, MAGE A3, MAGE A1, telomerase, PRAME, SSX2/4, PSCA, CTLA-4, gp100, GD2, GD3, fucosyl GM1, GM3, sLe (a), glycolipid F77, mesothelin, PD-L1, trp1, trp2, CD19, CD20, CD22, ROR1, CD33, c-Met, p53, p53 mutant, NY-ESO-1, PSMA, ETV6-AML, CEA, PSA, AFP, hTERT, EpCAM, ALK, androgen receptor, EphA2, CYP1B1, OY-TES-1, MAD-CT-2, MelanA/MART1, survivin, Ras, Ras mutant, ERG, bcr-abl, XBP1, etc. Examples of the viral antigens include, but are not limited thereto, inactivated viruses such as inactivated HBV and HPV, and proteins derived from various viruses such as EBV LMP1, EBV LMP2, EBNA (EBV nuclear antigen), HPV E1, HPV E2, HPV E6, HPV E7, HBV HBs, HTLV-1 Tax, and HBZ (HTLV-1 bZIP Factor), etc.

In one aspect of the present invention, an antigen recognized by a TCR transfected into cells that constitute a cell bank can be selected from the group consisting of GPC3, WT1, XAGE1, LMP2, NY-ESO-1, EB virus antigen and neoantigen, as well as peptide fragments thereof.

In one aspect of the present invention, an antigen recognized by a TCR transfected into cells that constitute a cell bank is EYILSLEEL, which is an HLA-A24-binding GPC3 peptide, FVGEFFTDV, which is an HLA-A2-binding GPC3 peptide, or a mixture thereof. In the present invention, an amino acid is represented in an abbreviation of conventional one-letter notation.

In the present invention, "having reactivity to a specific antigen" means that T cells react via TCR by specific binding/conjugation to epitope peptides derived from tumor-related antigens presented to major histocompatibility complex (MHC) class I or class II on antigen presenting cells, and refers to that the T cells do not react by binding/conjugation to other epitope peptides described above. Examples of the reactions via TCR of the T cells by binding/conjugation to epitope peptides derived from tumor-related antigens presented to MHC class I or class II include cytotoxicity, production of IFN-γ and granzyme, expression of T cell activation markers, and activation of transcription factors such as NF-AT.

In the present invention, a TCR transfected into cells that constitute a cell bank is collected from a subject who is a cancer patient or a non-cancer patient. A subject from whom T cells are collected may be a cancer patient who may have been administered, is currently administered, or is scheduled to be administered in the future a cancer vaccine, and may be a non-cancer patient to whom a cancer vaccine is not administered. One or more types of cancer vaccine may be administered. A cancer vaccine is a cancer or tumor-specific protein or peptide, a composition comprising a vaccine antigen derived from a cancer or said antigen, for inducing a cancer or tumor specific immune response. Usually, cancer vaccines contain adjuvants to enhance the specific immune response induced by the vaccine antigen. T cells are preferably αβ T cells. Preferable collection source of T cells is peripheral blood because of its low invasiveness, but is not limited thereto. Examples of other preferable collection sources include all collection sources in the body, such as cancer tissue or tumor tissue, lymph node or other tissue or organ, or blood, umbilical cord blood, lymph fluid, interstitial fluid (inter-tissue liquid, inter-cell liquid and interstitial fluid), body cavity fluid (ascitic fluid, pleural fluid, pericardial fluid, cerebrospinal fluid, synovial fluid, and aqueous humor). In one aspect of the present invention, preferable T cells are those derived from tumor tissue. T cells derived from tumor tissue are usually tumor infiltrating T cells.

In the case where the subject is a cancer patient, the cancer of the cancer patient is selected from ovarian cancer, hepatoblastoma, hepatoma, gastric cancer, esophageal cancer, pancreatic cancer, renal cell cancer, breast cancer, malignant melanoma, non-small cell lung cancer, cervical cancer, glioblastoma, prostate cancer, neuroblast, chronic lymphocytic leukemia, thyroid papilla cancer, colon cancer, or B cell non-Hodgkin's lymphoma. The cancer is preferably hepatoma or hepatoblastoma.

In the present invention, iPS cells may be prepared by reprogramming non-T non-B cells or monocytes. Besides, they may also be those already prepared from non-T non-B cells or monocytes. The "non-T non-B cells" mean mononuclear cells that are classified neither as T cells nor as B cells. Non-T non-B cells can be prepared by collecting peripheral blood mononuclear cells and then removing the T cells and B cells contained in the mononuclear cells. Peripheral blood mononuclear cells can be isolated from human whole blood by a mononuclear cell separation solution. Lymphoprep^{®}can be exemplified as a mononuclear cell separation solution. In order to remove B cells and T cells from mononuclear cells, magnetic beads such as flow cytometry or MACS^{®} beads may be used by utilizing antibodies against CD19, CD20, CD22 which are surface antigens of B cells, or B cell receptors, and CD3, CD4 or CD8 which are surface antigens of T cells.

"T cells" can be prepared as cells expressing CD3 and CD8 after collection as peripheral blood mononuclear cells. In order to purify T cells from peripheral blood mononuclear cells, magnetic beads such as flow cytometry or MACS^{®} beads may be used by utilizing antibodies against CD3, CD4 or CD8 which are surface antigens of T cells.

Methods for producing iPS cells are known in the art. In the present invention, iPS cells can be preferably induced by introducing cell reprogramming factors into non-T non-B cells, monocytes, or T cells. Examples of the cell reprogramming factors include genes such as Oct3/4, Sox2, Sox1, Sox3, Sox15, Sox17, klf4, klf2, c-Myc, N-Myc, L-Myc, Nanog, Lin28, Fbx15, ERas, ECAT15-2, Tcl1, β-catenin, Lin28b, Sall1, Sall4, Esrrb, Nr5a2, Tbx3, and Glis1, etc., or gene products. These cell reprogramming factors may be used alone or in combination. From the viewpoint of efficiently establishing iPS cells, among these cell reprogramming factors, it is preferable to introduce Oct3/4, Sox2, Klf4, and c-Myc (so-called Yamanaka's four factors) into the non-T non-B cells, monocytes, or T cells.

Methods for introducing cell reprogramming factors into the non-T non-B cells, monocytes, or T cells are not particularly limited, and methods known in the art can be adopted. For example, in a case of transfecting a gene encoding the cell reprogramming factor into the non-T non-B cells, monocytes, or T cells, a gene encoding the cell reprogramming factor (e.g., cDNA) can be inserted into an expression vector comprising a promoter functioning in the cells, and the expression vector can be transfected into the cells by infection or methods of lipofection, liposome and calcium phosphate coprecipitation, DEAE dextran, microinjection, or electroporation. In the case where the cell reprogramming factor is in the form of a protein and the protein is introduced into the non-T non-B cells, monocytes, or T cells, a method using a protein introduction reagent, a method using protein introduction domain fusion protein, electroporation method, and microinjection method can be exemplified. In the case where the cell reprogramming factor is in the form of a messenger RNA (mRNA) and the mRNA is transfected into the non-T non-B cells, monocytes, or T cells, a method using an mRNA transfection reagent and a method of addition to a culture medium can be exemplified.

Examples of expression vectors used for transfection by infection include virus vectors such as lentivirus, retrovirus, adenovirus, adeno-associated virus, herpes virus, and Sendai virus and animal cell expression plasmids. However, from the viewpoints of less possibility of insertional mutagenesis, high transfection efficiency, and large number of copies of transfected genes, Sendai virus is preferably used to transfect a gene encoding the cell reprogramming factor into the cells.

Examples of the promoters used in the expression vector for transfecting a gene encoding the cell reprogramming factor into cells include SRα promoter, SV40 promoter, LTR promoter, CMV promoter, RSV promoter, HSV-TK promoter, and ubiquitin promoter, etc. These promoters may be capable of controlling the expression of a gene inserted downstream of the promoter depending on the presence or absence of a drug such as tetracycline. The expression vector may comprise enhancer, poly A addition signal, selection marker gene (e.g., neomycin resistance gene), SV40 replication origin, etc., in addition to promoters.

A culture medium used for culturing iPS cells obtained by reprogramming is not particularly limited. It can be prepared by using a culture medium for culturing animal cells as a basal culture medium, and adding thereto cytokines for maintaining undifferentiation potential of iPS cells. Examples of basal culture media include Iscove's Modified Dulbecco's Medium (IMDM), Medium 199, Eagle's Minimum Essential Medium (EMEM), αMEM medium, Dulbecco's Modified Eagle's Medium (DMEM), Ham's F12 medium, RPMI 1640 medium, Fischer's Neurobasal Medium (Life Technologies), StemFit^{®} AK03N (Ajinomoto Healthy Supply), and a mixed medium thereof. A culture medium may be added with serum or may be serum-free. bFGF may be exemplified as a preferable cytokine, and the concentration thereof in a culture medium is, for example, 1 to 100 µg/mL (preferably 50 µg/mL).

In one aspect of the present invention, the method for culturing iPS cells may be adhesion culture or suspension culture, preferably adhesion culture. Examples of the methods for isolating iPS cells include physical methods using a cell scraper or the like, and methods using a dissociation solution with protease activity, or collagenase activity, or protease activity and collagenase activity (e.g., Accutase^{®} and Accumax^{®}, etc.).

In one aspect of the present invention, iPS cells are preferably subcultured in another incubator when they reach a cell density of 1×10³ to 1×10⁴ cells/cm², 1×10⁴ to 1×10⁵ cells/cm², or 1×10⁵ to 1×10⁶ cells/cm². The passage number may be any number as long as a required amount of iPS cells can be obtained, preferably 1 to 5 or 5 to 10.

iPS cells that have been introduced with Yamanaka's four factors and reprogrammed consist of a large number of iPS cell clones. As will be described later, in order to select iPS cell clones with good differentiation efficiency into T cells, it is preferable to clone iPS cells by performing colony picking. Methods for colony picking are not particularly limited. A method using pipetman under a microscope, limiting dilution analysis, a method using fully automatic colony picker are used. Preferably, one or more kinds, preferably three or more kinds, more preferably six or more kinds of resulting iPS cell clones are stored, and constitute a cell bank. As cell banks, it is preferable to cryopreserve iPS cell clones. Methods of cryopreservation of cells are well known to those skilled in the art. For example, the cultured iPS cell clones can be harvested and washed with a buffer solution or a culture medium, enriched by centrifugation or etc. after the cell number is counted, suspended in a medium for cryopreservation (e.g., a culture medium containing 10% DMSO), and then cryopreserved at a low temperature. In another aspect, an established iPS cell clone with good differentiation efficiency into T cells may be used.

"Differentiation efficiency" refers to the abundance ratio of hematopoietic stem cells, immature T cells and mature T cells, respectively in all viable cells in each differentiation stage from iPS cells into hematopoietic stem cells, from hematopoietic stem cells into immature T cells, and from immature T cells into mature T cells. Identification of differentiated cells in each differentiation stage can be performed by FACS analysis of surface markers. The differentiation efficiency into hematopoietic stem cells is indicated as the ratio of CD34/CD43 double-positive cells in all viable cells. The differentiation efficiency into immature T cells is indicated as the ratio of CD4/CD8 double-positive cells or that of CD5 positive cells in all viable cells. In addition, the differentiation efficiency into mature T cells is indicated as the ratio of the cells wherein CD8 α chain, CD8 β chain, TCR α chain, and TCR β chain are all positive in all viable cells.

"Differentiation efficiency is good." refers to that the ratio of CD34/CD43 double-positive cells, which are hematopoietic stem cells, is 5 to 15% or more in the differentiation from iPS cells into hematopoietic stem cells, the ratio of CD4/CD8 double-positive cells or that of CD5 positive cells, which are immature T cells, is 10% or more or 50% or more, respectively in the differentiation from hematopoietic stem cells into immature T cells, and the ratio of the cells wherein CD8 α chain, CD8 β chain, TCR α chain and TCR β chain are all positive is 50% or more in the differentiation from immature T cells into mature T cells.

The mature T cells of the present invention are preferably produced by firstly differentiating the iPS cell clones into hematopoietic stem cells, then differentiating the hematopoietic stem cells into immature T cells, and finally differentiating immature T cells into CD8 single-positive T cells. The immature T cells of the present invention are preferably produced by first differentiating the iPS cell clones into hematopoietic stem cells and then differentiating the hematopoietic stem cells into immature T cells. The hematopoietic stem cells of the present invention are preferably produced by differentiating the iPS cell clones into hematopoietic stem cells.

### Culture of hematopoietic stem cells and immature T cells

Hematopoietic stem cells are preferably produced by culturing iPS cells in a culture medium added with vitamin C. Here, "vitamin C" refers to L-ascorbic acid and a derivative thereof. A "derivative of L-ascorbic acid" means one that is converted into vitamin C by an enzymatic reaction *in vivo.* Examples of derivatives of L-ascorbic acid include vitamin C phosphate, ascorbyl glucoside, ethyl ascorbic acid, vitamin C esters, ascorbyl tetrahexyldecanoate, ascorbyl stearate, and ascorbic acid-2-phosphate-6-palmitate. A preferable derivative of L-ascorbic acid is vitamin C phosphate, and examples thereof include phosphoric acid-L-ascorbates such as phosphoric acid-sodium L-ascorbate or phosphoric acid-magnesium L-ascorbate. Vitamin C is contained in a culture medium at a concentration of, for example, 5 to 500 µg/mL.

A culture medium used for producing hematopoietic stem cells is not particularly limited. It can be prepared by using a culture medium for culturing animal cells as a basal culture medium, and adding vitamin C, etc. thereto. Examples of basal culture media include Iscove's Modified Dulbecco's Medium (IMDM), Medium 199, Eagle's Minimum Essential Medium (EMEM), αMEM medium, Dulbecco's Modified Eagle's Medium (DMEM), Ham's F12 medium. RPMI 1640 medium, Fischer's medium, Neurobasal Medium (Life Technologies), StemPro34 (Life Technologies), and a mixed medium thereof. A culture medium may contain serum or may be serum-free. If necessary, a basal medium may contain one or more substances selected from, for example, albumin, insulin, transferrin, selenium, fatty acids, trace elements, 2-mercaptoethanol, thioglycerol, monothioglycerol, lipids, amino acids, L-glutamine, non-essential amino acids, vitamins, growth factors, low-molecular-weight compounds, antibiotics, antioxidants, pyruvic acid, buffers, inorganic salts, and cytokines, etc.

A cytokine selected from the group consisting of BMP4 (bone morphogenetic protein 4), VEGF (vascular endothelial growth factor), bFGF (basic fibroblast growth factor), SCF (stem cell factor), TPO (thrombopoietin), and FLT3L (Flt3 ligand) may be further added to a culture medium used for producing hematopoietic stem cells. The concentrations thereof are, for example, 1 to 100 ng/mL for BMP4, 1 to 100 ng/mL for VEGF, 1 to 100 ng/mL for bFGF, and 10 to 100 ng/mL for SCF, 1 to 100 ng/mL for TPO, and 1 to 100 ng/mL for FLT3L.

A TGF β inhibitor may be added to a culture medium of hematopoietic stem cells. A "TGF β inhibitor" is a low-molecular inhibitor that interferes with signaling of TGF β family. Examples thereof include SB431542 and SB202190 (R. K. Lindemann et al., Mol. Cancer 2: 20 (2003)), SB505124 (GlaxoSmithKline), NPC30345, SD093, SD908, and SD208 (Scios), as well as LY2109761, LY364947, and LY580276 (Lilly Research Laboratories). The concentration thereof in a culture medium is preferably 0.5 to 100 µM.

iPS cells may be co-cultured with feeder cells such as C3H10T1/2 (Takayama N., et al. J Exp Med. 2817-2830, 2010), or heterologous stromal cells (Niwa A et al. J Cell Physiol. 2009 Nov; 221 (2): 367-77), etc.

The method for culturing iPS cells in the production of hematopoietic stem cells may be adhesion culture or suspension culture. However, suspension culture is preferable. For example, iPS cells can be subjected to suspension culture after releasing colonies cultured to 80% confluency in a dish used and dissociating them into single cells. Examples of the methods for isolating iPS cells include physical methods using a cell scraper or the like, and methods using a dissociation solution with protease activity and collagenase activity (e.g., Accutase^{®} and Accumax^{®}, etc.), or a dissociation solution with collagenase activity.

Suspension culture is a way of culturing cells wherein the cells do not adhere to a culture vessel. Suspension culture can be performed by using a culture vessel which is not artificially treated (e.g., coating with an extracellular matrix, etc.) for improving adhesion with cells, or a culture vessel which is artificially treated to suppress adhesion (e.g., coating with polyhydroxyethyl methacrylate (poly-HEMA) or nonionic surfactant polyol (Pluronic F-127, etc.). However, it is not particularly limited. During a suspension culture, it is preferable to form and culture embryoid bodies (EB). When suspension-culturing embryoid bodies to obtain hematopoietic stem cells, it is preferable to dissociate them into single cells and then perform the adhesion culture.

Hematopoietic stem cells can also be prepared from cystoid-like structures (also referred to as iPS-sac) obtained by culturing iPS cells. Here, the "cystoid-like structure" is an iPS cell-derived three-dimensional bursal structure (with internal space), formed in population of endothelial cells etc, and containing hematopoietic stem cells inside.

The temperature condition during culture for producing hematopoietic stem cells from iPS cells is not particularly limited, but for example about 37°C to about 42°C, and preferably about 37°C to about 39°C. In addition, the culture period can be appropriately determined by a person skilled in the art while monitoring the number of hematopoietic stem cells etc. The number of culturing days is not particularly limited as long as hematopoietic stem cells can be obtained, but is, for example, at least 6 days or more, 7 days or more, 8 days or more, 9 days or more, 10 days or more, 11 days or more, 12 days or more, 13 days or more, or 14 days or more, and preferably 14 days. A long culture period does not cause any problem in the production of hematopoietic stem cells. Besides, a low-oxygen condition is also possible. In one aspect of the present invention, examples of oxygen concentration as low-oxygen conditions include 15%, 10%, 9%, 8%, 7%, 6%, 5%, or less.

"CD4/CD8 double-positive T cells" are the cells among T cells wherein the surface antigens CD4 and CD8 are both positive (CD8⁺ CD4⁺). Since the T cells can be recognized by the fact that surface antigens CD3 and CD45 are positive, CD4/CD8 double-positive T cells can be identified as the cells wherein CD4, CD8, CD3, and CD45 are positive. CD4/CD8 double-positive T cells can be differentiated into CD4 single-positive cells or CD8 single-positive cells by induction.

CD4/CD8 double-positive T cells can be produced by a method which comprises a step of culturing hematopoietic stem cells in a culture medium added with p38 inhibitor and/or SDF-1.

A "p38 inhibitor" is defined as a substance that inhibits the function of p38 protein (p38MAP kinase). Examples of p38 inhibitors include a chemical inhibitor of p38, a dominant negative mutant of p38, or a nucleic acid encoding the same, however, they are not limited thereto.

Examples of chemical inhibitors of p38 include SB 203580 (4-(4-fluorophenyl)-2-(4-methylsulfonylphenyl)-5-(4-pyridyl)-1H-imidazole) and derivatives thereof, SB202190 (4-(4-fluorophenyl)-2-(4-hydroxyphenyl)-5-(4-pyridyl)-1H-imidazole) and derivatives thereof, SB239063 (trans-4-[4-(4-fluorophenyl)-5-(2-methoxy-4-pyrimidinyl)-1H-imidazole-1-yl] cyclohexanol) and derivatives thereof, SB220025 and derivatives thereof, PD169316, RPR200765A, AMG-548, BIRB-796, SCIO-469, SCIO-323, VX-702, or FR167653, however, they are not limited thereto. These compounds are commercially available. For example, SB203580, SB202190, SC239063, SB220025, and PD169316 are available from Calbiochem, and SCIO-469 and SCIO-323 are available from Scios etc. A p38 inhibitor is added to a culture medium in a range of, for example, about 1 µM to about 50 µM.

Examples of dominant negative mutants of p38 include p38T180Ain which threonine at position 180 located in the DNA binding region of p38 is point-mutated to alanine, and p38Y182F in which tyrosine at position 182 of p38 in human and mouse is point-mutated to phenylalanine, and the like.

SDF-1 (stromal cell-derived factor 1) may be not only SDF-1α or its mature form, but also isoforms such as SDF-1β, SDF-1γ, SDF-1δ, SDF-1ε, or SDF-1ϕ, or mature forms thereof, or a mixture thereof at any proportion. Preferably, SDF-1α is used. SDF-1 may also be referred to as CXCL-12 or PBSF.

SDF-1 may be substituted, deleted and/or added with one or several amino acids in its amino acid sequence as long as having the activity as the chemokine. Similarly, sugar chains may be substituted, deleted and/or added. An amino acid mutation is acceptable if at least four cysteine residues (Cys30, Cys32, Cys55, and Cys71 in case of human SDF-1α) are retained in SDF-1, and it has the identity of 90% or more to the amino acid sequence of natural form. SDF-1 may be that of a mammal such as a human, a non-human mammal such as monkey, sheep, bovine, horse, pig, dog, cat, rabbit, rat, or mouse, etc. For example, the protein registered in GenBank with registration number of NP_954637 can be used as human SDF-1α, and the protein registered in GenBank with registration number of NP_000600 can be used as SDF-1β.

As SDF-1, those which are commercially available may be used, those which are purified from natural products may be used, alternatively, those which are produced by peptide synthesis or genetic engineering techniques may also be used. SDF-1 is added to a culture medium in a range of, for example, about 10 ng/mL to about 100 ng/mL.

A culture medium used for producing CD4/CD8 double-positive T cells is not particularly limited. It can be prepared by using a culture medium for culturing animal cells as a basal culture medium, and adding p38 inhibitor and/or SDF-1, and furtherly vitamin C. The types of vitamin C used in the production of CD4/CD8 double-positive T cells are, for example, as described above, and the concentration thereof is, for example, 5 to 200 µg/mL. Examples of basal culture media include Iscove's Modified Dulbecco's Medium (IMDM), Medium 199, Eagle's Minimum Essential Medium (EMEM), αMEM medium, Dulbecco's Modified Eagle's Medium (DMEM), Ham's F12 medium, RPMI 1640 medium, Fischer's Neurobasal Medium (Life Technologies), and a mixed medium thereof. A culture medium may be added with serum or may be serum-free. If necessary, a basal medium may contain one or more substances selected from, for example, albumin, insulin, transferrin, selenium, fatty acids, trace elements, 2-mercaptoethanol, thioglycerol, lipids, amino acids, L-glutamine, non-essential amino acids, vitamins, growth factors, low-molecular-weight compounds, antibiotics, antioxidants, pyruvic acid, buffers, inorganic salts, and cytokines, etc.

A cytokine selected from the group consisting of SCF, TPO (thrombopoietin), FLT3 L, and IL-7 may be further added to a culture medium used for producing CD4/CD8 double-positive T cells. The concentrations thereof are, for example, 10 to 100 ng/mL for SCF, 10 to 200 ng/mL for TPO, 1 to 100 ng/mL for FLT3L, and 1 to 100 ng/mL for IL-7.

Hematopoietic stem cells may be cultured by adhesion culture or suspension culture. However, adhesion culture is preferable. In the case of adhesion culture, a culture vessel may be coated and used. Examples of coating agents include Matrigel (Niwa A, et al. PLos One. 6 (7): e22261, 2011), collagen, gelatin, laminin, heparan sulfate proteoglycan, retronectin, Fc-DLL4 or entactin, and combinations thereof.

The condition of culture temperature for culturing hematopoietic stem cells to produce CD4/CD8 double-positive T cells is not particularly limited, but preferably about 37°C to about 42°C, and more preferably about 37°C to about 39°C. In addition, the culture period can be appropriately determined by a person skilled in the art while monitoring the number of CD4/CD8 double-positive T cells or the like. The number of culturing days is not particularly limited as long as CD4/CD8 double-positive T cells can be obtained, but is, for example, at least 10 days or more, 12 days or more, 14 days or more, 16 days or more, 18 days or more, 20 days or more, 22 days or more, or 23 days or more, and preferably 23 days.

The resulting CD4/CD8 double-positive T cells may be isolated and used, or used as a cell population containing other cell species. In the case of isolation, methods well known to those skilled in the art can be used. For example, a method of labeling with antibodies against CD4, CD8, CD3 and/or CD45, and isolating using a flow cytometer, or a purification method using an affinity column in which a desired antigen is immobilized can be exemplified.

"CD8 single-positive T cells" are the cells among T cells wherein the surface antigen CD8 is positive (CD8⁺ CD4⁻), and are also referred to as cytotoxic T cells. Since the T cells can be recognized by the fact that surface antigens CD3 and CD45 are positive, CD8 single-positive T cells can be identified as the cells wherein CD8, CD3 and CD45 are positive while CD4 is negative.

CD8 single-positive T cells can be produced by a method comprising a step of culturing CD4/CD8 double-positive T cells in a culture medium added with a corticosteroid.

The corticosteroid is preferably a glucocorticoid or a derivative thereof, and examples thereof include cortisone acetate, hydrocortisone, fludrocortisone acetate, prednisolone, triamcinolone, methylprednisolone, dexamethasone, betamethasone, or beclomethasone dipropionate. Preferably, the corticosteroid is dexamethasone. The concentration thereof in a culture medium is, for example, 1 to 100 nM.

A culture medium used for producing CD8 single-positive T cells is not particularly limited. It can be prepared by using the culture medium for culturing animal cells as a basal culture medium, and adding a corticosteroid thereto. Examples of basal culture media include Iscove's Modified Dulbecco's Medium (IMDM), Medium 199, Eagle's Minimum Essential Medium (EMEM), αMEM medium, Dulbecco's Modified Eagle's Medium (DMEM), Ham's F12 medium, RPMI 1640 medium, Fischer's Neurobasal Medium (Life Technologies), and a mixed culture medium thereof. A culture medium may be added with serum or may be serum-free. If necessary, a basal medium may contain one or more substances selected from, for example, albumin, insulin, transferrin, selenium, fatty acids, trace elements, 2-mercaptoethanol, thioglycerol, monothioglycerol, lipids, amino acids, L-glutamine, non-essential amino acids, vitamins, growth factors, low-molecular-weight compounds, antibiotics, antioxidants, pyruvic acid, buffers, inorganic salts, and cytokines, etc.

The culture medium used in the production of CD8 single-positive T cells preferably further contains anti-CD3 antibody, vitamin C, or cytokine. Examples of the cytokine include IL-2, IL-7, IL-15, IL-21, and the like.

The anti-CD3 antibody is not particularly limited as long as it is an antibody that specifically recognizes CD3. Examples thereof include antibodies produced from OKT3 clones. The concentration of the anti-CD3 antibody in a culture medium is, for example, 10 to 1000 ng/mL.

The vitamin C used in the production of CD8 single-positive T cells is, for example, those described above, and can be used in the same conditions as described above.

The concentrations of cytokines in a culture medium used in the production of CD8 single-positive T cells are, for example, 10 to 1000 U/mL for IL-2 and 1 to 100 ng/mL for IL-7.

The temperature condition during the culture of CD4/CD8 double-positive T cells for producing CD8 single-positive T cells is not particularly limited, but for example, preferably about 37°C to about 42°C, and more preferably about 37°C to about 38°C. In addition, the culture period can be appropriately determined by a person skilled in the art while monitoring the number of CD8 single-positive T cells etc. The number of culturing days is not particularly limited as long as CD8 single-positive T cells can be obtained, but is, for example, at least 1 day or more, 2 days or more, 3 days or more, 4 days or more, or 5 days or more, and preferably 3 days.

### Preparation of cDNA encoding TCR

In the present invention, the preparation of cDNAs encoding TCR α chains and β chains that are transfected into cells that constitute a cell bank is preferably performed for each single cell. T cells collected from a subject are T cell populations having genetic diversity as a whole. However, the antigen or peptide sequence recognized by the TCR of individual T cells is determined and the antigen specificity of individual T cells is different. In order to select the TCR that is optimal for each tumor-related antigen, that is, the TCR that has high reactivity to each tumor-related antigens, it is preferable to prepare cDNAs for each single cell.

### Preparation of cDNA encoding CAR

In the present invention, a cDNA encoding a CAR transfected into cells that constitute a cell bank is prepared by constructing in plasmid vector or viral vector, a gene in which a target binding site of an antibody or phage or ligand or receptor which bind to a target cell surface antigen is linked to an intracellular signal site in a CD3 zeta gene and CD28 or CD137 via a linker and a cell membrane penetration site (e.g., a transmembrane site of a CD8 molecule).

It is preferable to culture and proliferate the T cells obtained from a subject with a targeted tumor-related antigen. Activation markers may be used from T cell populations responsive to the tumor-related antigen used to isolate single cells by a cell sorter, for example. As the activation marker, cell surface CD137 is preferable. Known techniques for isolating human T cells include, for example, flow cytometry using antibodies to T cell surface markers, such as CD3 and CD137, and cell sorters. The obtained single T cells can be subjected to gene cloning using PCR methods to proliferate cDNAs encoding TCR α and β chains, respectively.

### Isolation of TCR gene by single cell PCR

In order to obtain single cells, single cell sorting by a cell sorter can be performed by binding CD8-positive T cells specific to tumor antigens obtained from peripheral blood or the like to MHC Dextramer^{®} that forms a complex with antigen peptide. The MHC dextramer is a compound composed of a dextran polymer skeleton in which MHC and fluorescent dye molecules are bound. In place of MHC dextramer, MHC tetramer may be used. The MHC tetramer is a tetramer of a complex of an antigen peptide and an MHC molecule with biotin and avidin. In another aspect, tumor antigen-specific CD8-positive T cells obtained from peripheral blood or the like are cultured in the presence of the antigen and proliferated, and CD3/CD137 double-positive cells can then be sorted for single cells by a cell sorter. In another aspect, from CD3/CD137 double-positive cells, a cell population that binds to MHC dextramer complexed with the antigenic peptide can be sorted for single cells by a cell sorter. RNA can be extracted from the single cells obtained, and the TCR gene pair (TCR α chain gene and TCR β chain gene) can be isolated by a PCR method using the cDNAs obtained by reverse transcription. Sequence analysis of the isolated TCR gene pair can be performed, and the type of tumor antigen-reactive T cells (TCR repertoire) and its appearance frequency can be analyzed.

### Construction of vectors

The PCR fragment amplified using the cDNA of isolated TCR as a template can be incorporated into a viral vector or a non-viral vector (transposon vector) using, for example, Gibson Assembly System. Specifically, a gene in which the isolated TCR α chain gene and TCR β chain gene are linked via the T2A sequence is linked downstream of the ubiquitin promoter. Further downstream thereof, EGFR (EGFRt, truncated EGFR) excluding the ligand-binding site and intracellular domain for the IRES (internal ribosome entry site) sequence or a marker gene such as CD19 lacking an intracellular domain are linked. The construct is incorporated into a viral vector or a non-viral vector. As the vector, a viral vector and a non-viral vector can be used. However, a non-viral vector is preferable. As the non-viral vector, among transposon vectors, a piggyBac^{®} vector is preferable. Compared to conventional viral vector method, the transposon method is an inexpensive and safe transfection method of next generation.

### Transfection of TCR cDNAs into cells

As a method of transfecting cDNA pairs respectively encoding TCR α and β chains from non-T non-B cells or monocyte-derived iPS cell clones with good differentiation into T cells, the hematopoietic stem cells differentiated from the iPS cell clones or the immature T cells differentiated from the hematopoietic stem cells, either a method using a viral vector or a non-viral vector can be adopted. Examples of the viral vectors include viral vectors such as lentivirus, retrovirus, adenovirus, adeno-associated virus, herpes virus and Sendai virus, etc. and animal cell-expressing plasmid. However, retrovirus or lentivirus are preferable. When a retroviral or a lentiviral infection is carried out, a spin infection method or the like is preferably used. When a non-viral vector is used, a transposon method is preferable. Examples of transfection methods of non-viral vector include lipofection method, liposome method, calcium phosphate coprecipitation method, DEAE dextran method, microinjection method, and electroporation method. PCR products can be directly transfected into cells without using a vector. An electroporation method is preferably used for transfecting transposon vectors or PCR products into cells. As a electroporation apparatus, the transfection device of ExPERT^{®}system (MaxCyte) is preferable.

Examples of the promoters used in the expression vector for transfecting the cDNA pair into the cells include EF1α promoter, SRα promoter, SV40 promoter, LTR promoter, CMV promoter, RSV promoter, HSV-TK promoter, ubiquitin promoter, and the like. These promoters may be capable of controlling the expression of genes inserted downstream of the promoter depending on the presence or absence of a drug such as tetracycline. An expression vector can comprise enhancer, poly A addition signal, selection marker gene (e.g., neomycin resistance gene), SV40 replication origin, etc., in addition to the promoter.

### Transfection of TCR into mature T cells using genome editing techniques

The transfection of TCR into mature T cells can be performed using genome editing techniques. For gene sequence of targeted endogenous TCR α and β chains, the sequence including its upstream is confirmed and a guide RNA (guide RNA for the sense strand and guide RNA for the antisense strand) that has a high cleavage activity for the endogenous TCR α and β chains gene and does not cleave the sequence on the genome except the target gene is produced. In parallel, donor DNA that comprises the homologous recombination sites upstream and downstream of the insertion site of the TCR α and β chains to be transfected is produced. A TCR of interest can be transfected by transfections of the guide RNA, Cas9 and the donor DNA into mature T cells, which can be carried out by using viral vectors, non-viral vectors, electroporation, and the like. Mature T cells in which the endogenous TCR is replaced by a TCR of interest can be selected using binding to a targeted tumor-related antigen as an indicator.

The hematopoietic stem cell differentiated from the iPS cell clone into which the cDNA pair has been transfected, the immature T cell differentiated from the hematopoietic stem cell, and the mature T cell are cultured under culture conditions such as the culture solution, the culture solution composition, and the culture temperature described above, whereby a regenerated T cell expressing a TCR comprising an α chain and a β chain respectively encoded by the cDNA pair is obtained. Whether or not the targeted TCR is expressed can be confirmed, for example, based on the repertoire analysis of TCR β gene, using a TCRV β analysis kit (Beckman Coulter, catalog number IM-3497). Since a marker gene such as EGFRt (truncated EGFR) and CD19t (truncated CD19) is incorporated into a vector, expression of the TCR α and β chains can be speculated by analyzing the expression of the marker gene.

### Production of regenerated T cells

The production of regenerated T cells can be initiated by transfecting a TCR pair having reactivity to tumor-related antigens into mature T cells, immature T cells or hematopoietic stem cells that constitute a cell bank of the present invention. Therefore, it is possible to rapidly produce regenerated T cells for use in prevention and/or treatment of cancer or infectious diseases.

### T cells as TCR acquisition source

T cells which are acquisition source of TCR pairs corresponding to tumor-related antigens are preferably collected from tumor tissue or peripheral blood of patients. In a case where it is necessary to previously administer a tumor-related antigen to a subject, in order to cause tumor-related antigen-specific T cells to appear in the tumor tissue or peripheral blood, it is preferable to collect T cells from tumor tissue or peripheral blood after the administration of the tumor-related antigen to the subject. As a tumor-related antigen like this, GPC3 can be exemplified. On the other hand, even when a tumor-related antigen is not administered, and tumor-specific T cells are present in peripheral blood, T cells specific to tumor-related antigen may be isolated from peripheral blood using, for example, an MHC dextramer, without performing the administration of a tumor-related antigen. Examples of such tumor-related antigens include WT1, NYESO-1, EBV antigen, and the like.

As described above, the T cells collected from tumor tissue or peripheral blood of a subject either after administration of a tumor-related antigen or without the administration of a tumor-related antigen are preferably stimulated by the tumor-related antigen in a test tube, and then isolated using an activation marker after proliferating the cell population which is responsive to tumor. In the case where the frequency of appearance of T cells specific to a tumor-related antigen is extremely high in the peripheral blood, stimulation by the tumor-related antigen in a test tube may not be performed.

When a subject from whom T cells for obtaining TCR pairs are collected, and a subject who is to be treated for cancer with a replenishment therapy using regenerated T cells into which the obtained TCR has been transfected are separate individuals, it is preferable to confirm that the TCR pair does not induce an alloreaction to the cells of the subject who is to be treated for cancer.

### Pharmaceutical compositions

Pharmaceutical compositions containing regenerated T cells produced by transfecting an antigen-specific TCR into differentiated cells derived from the iPS cells of the present invention can be used in the treatment of cancer and infectious diseases. The pharmaceutical compositions of the present invention can be produced by methods commonly used in the field of formulation technology, such as those described in the Japanese Pharmacopoeia. The pharmaceutical compositions of the present invention may contain pharmaceutically acceptable additives. Examples of the additives include a cell culture medium, physiological saline, a suitable buffer (e.g., phosphate buffer), and the like.

The pharmaceutical compositions can be produced by suspending regenerated T cells in physiological saline or a suitable buffer (e.g., phosphate buffer). In order to exhibit a desired therapeutic effect, one dose preferably contains, for example, 1×10⁷ or more cells. More preferably, the content of the cells is 1×10⁸ or more, and further more preferably 1×10⁹ or more. The content of the cells can be appropriately adjusted in consideration of sex, age, body weight of the subject, state of affected part and the cells, etc. For protecting the cells, the pharmaceutical composition may contain dimethyl sulfoxide (DMSO) and serum albumin etc., in addition to regenerated T cells as active ingredients. Moreover, for preventing contamination by bacteria, they may contain antibiotics etc. and vitamins, cytokines, etc. in order to promote activation and differentiation of cells. Furthermore, the pharmaceutical compositions may contain other pharmaceutically acceptable ingredients (for example, carrier, excipient, disintegrant, buffer, emulsifier, suspension, analgesic agent, stabilizer, preservative, antiseptic agent, physiological saline, etc.).

The hematopoietic stem cells, immature T cells or mature T cells that constitute the cell bank of the present invention can be dispensed depending on the amount of use in one TCR transfection (1 × 10⁷ to 1 × 10⁹) and cryopreserved. In case of cryopreservation, the storage temperature is not particularly limited as long as it is suitable for the preservation of cells. Examples thereof include -150°C to -196°C, preferably -150°C or lower. In case of cryopreservation, cells are preferably preserved in an appropriate container such as a vial and a bag for cryopreservation. Procedures for minimizing the risk of cell damage during freezing and thawing of regenerated T cells are well known to those skilled in the art.

Quantity of stock of the cells that constitute the cell bank of the present invention is preferably that which allows for TCR transfection to be performed 1000 times or more.

The pharmaceutical compositions are used for prevention and/or treatment of cancer or infectious diseases. Examples of cancer include, but are not limited to, ovarian cancer, hepatoblastoma, hepatoma, gastric cancer, esophageal cancer, pancreatic cancer, renal cell cancer, breast cancer, malignant melanoma, non-small cell lung cancer, cervical cancer, glioblastoma, prostate cancer, neuroblast, chronic lymphocytic leukemia, thyroid nipple cancer, colon cancer, and B cell non-Hodgkin's lymphoma.

### EXAMPLES

The present invention will be described below according to examples. However, the present invention is not limited to the following examples.

### Example 1

### Use (1) for production of regenerated T cells of a cell bank constructed by differentiated cells produced from iPS cells

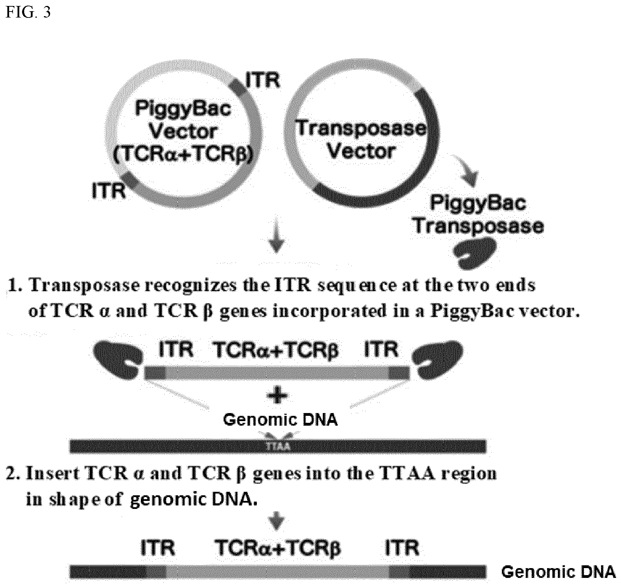

FIG. 1 shows steps of producing antigen-specific regenerated T cells from mature T cells that constitute the cell bank of the present invention. A TCR gene isolated from a patient-derived T cell that specifically reacts with tumor antigen X, a TCR gene specific to an established tumor antigen X or a chimeric receptor gene specific to tumor antigen X is transfected into mature T cells that constitute a cell bank. It takes about two weeks to produce regenerated T cells from mature T cells that constitute a cell bank. Since regenerated T cells for use in the treatment of cancer can be produced in said period, it is possible to reselect a TCR gene that is effective for treatment even when tumor recurrence or mutation of tumor cell occurs in patients undergoing cancer treatment. For the transfection of TCR genes or CAR genes specific to tumor antigen X, genome editing using CRISPR/Cas9 etc. or lentiviral or transposon vectors can be used. FIG. 3 shows steps of transfecting a TCR gene using a transposon.

Mature T cells produced from iPS cells were used to construct the cell banks of the invention which are assembly of frozen cells. The mature T cells are the T cells which recognize single antigens and do not cause an alloreaction. After thawing the frozen cells that constitute the cell bank, a TCR specific to tumor is transfected.

In the cell bank of the present invention, it is possible to produce regenerated T cells by transfecting a TCR gene that reacts with tumor antigen X into the mature T cells that are differentiated and induced from iPS cells and previously cryopreserved. For this reason, tumor-specific regenerated T cells can be produced in a shorter period of time (about two weeks) compared to the sequential steps of producing iPS cells, differentiating and inducing from iPS cells to mature T cells, and producing regenerated T cells by the transfection of TCR genes into mature T cells. Therefore, administration of tumor-specific regenerated T-cells to a patient can be performed quickly.

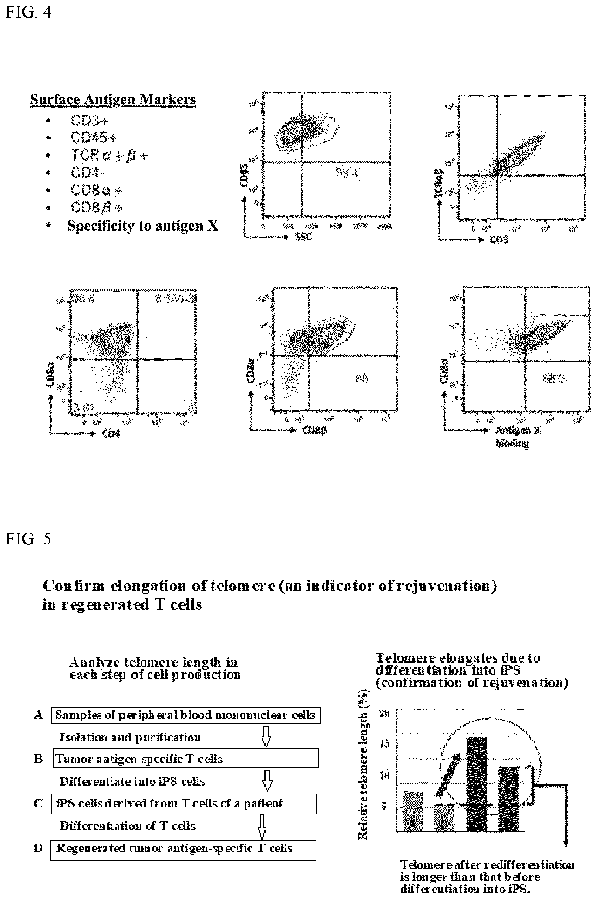

The phenotype was analyzed for the mature T cells (tumor antigen-specific CD8 positive cytotoxic T cells) induced from iPS cells as described above. Analysis results from the flow cytometer of regenerated T cells by cell surface antigen markers are shown in FIG. 4. In the mature T cells induced from IPS cells, expression of CD45+ TCR αβ+ CD3+ CD4- CD8 αβ+ which is expressed *in vivo* in mature cytotoxic T cells was confirmed.

Results of analyzing telomere, a cell aging marker (an indicator of rejuvenation) regarding the mature T cells that are differentiated and induced from iPS cells and constitute the cell bank of the present invention are shown in FIG. 5. Compared with the telomere length of the peripheral blood mononuclear cells of a patient (A) and the tumor antigen-specific T cells before regeneration (B), the telomere length of the iPS cells produced from the tumor antigen-specific T cells (C) was about 3 times, and that of the regenerated T cells of the present invention which were specific to tumor antigen (D) was about 2 times, and recovery in telomere length was confirmed in (C) and (D). That is, the improvement of cell aging is indicated in the regenerated T cells produced from the cells that constitute the cell bank of the present invention.

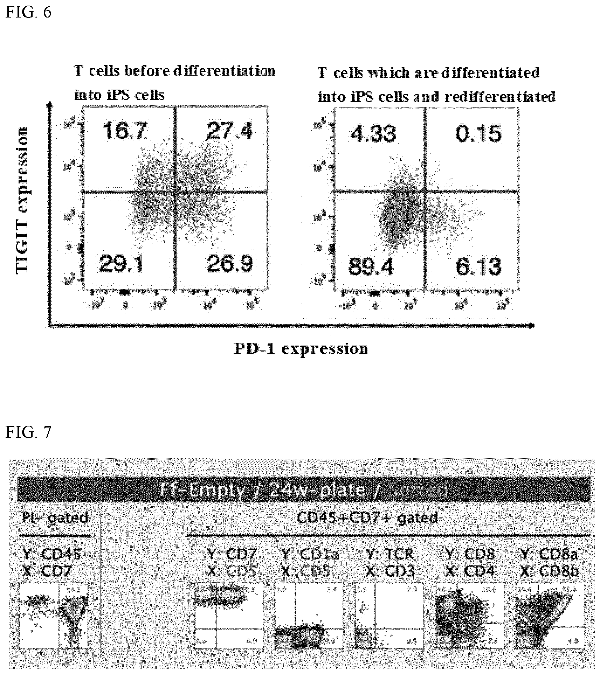

The mature T cells that are differentiated and induced from iPS cells and constitute the cell bank of the present invention were stained with anti-TIGIT antibody and anti-PD-1 antibody for the expression of PD-1, which was one of the cell exhaustion markers associated with immune checkpoints, and TIGIT molecule, and analyzed with a flow cytometer. For comparison, the tumor antigen-specific T cells before regeneration were also analyzed. The results are shown in FIG. 6. It was confirmed that the regenerated T cells of the present invention greatly reduced the expressions of PD-1 and TIGIT molecule compared to the tumor antigen-specific T cells before regeneration. Therefore, it is indicated that the regenerated T cells of the present invention have high cytotoxic activity.

### Example 2

### Use (2) for production of regenerated T cells of a cell bank constructed by differentiated cells produced from iPS cells

FIG. 2 shows steps of producing antigen-specific regenerated T cells from immature T cells that constitute the cell bank of the present invention. A TCR gene isolated from a patient-derived T cell that specifically reacts with tumor antigen X, a TCR gene specific to an established tumor antigen X or a chimeric receptor gene specific to tumor antigen X is transfected into immature T cells that constitute a cell bank. Even when tumor recurrence or tumor cell mutation occurs in patients undergoing treatment of cancer, it is possible to reselect a TCR gene that is effective for treatment. It takes about four weeks to produce regenerated T cells from immature T cells that constitute a cell bank. For the transfection of TCR genes or CAR genes specific to tumor antigen X, genome editing using CRISPR/Cas9 etc. or lentiviral or transposon vectors can be used.

Immature T cells produced from iPS cells were used to construct the cell banks of the invention which are assembly of frozen cells. The immature T cells are the T cells which do not express a T cell receptor. After thawing the frozen cells that constitute the cell bank, a TCR specific to tumor is transfected.

In the cell bank of the present invention, it is possible to produce regenerated T cells by transfecting a TCR gene that reacts with tumor antigen X into the immature T cells that are differentiated and induced from iPS cells and previously cryopreserved. For this reason, tumor-specific regenerated T cells can be produced in a shorter period of time (about four weeks) compared to the sequential steps of producing iPS cells, differentiating and inducing from iPS cells to immature T cells, and producing regenerated T cells by the transfection of TCR genes into immature T cells. Therefore, administration of tumor-specific regenerated T-cells to a patient can be performed quickly.

The phenotype was analyzed for the immature T cells (tumor antigen-specific CD8 positive cytotoxic T cells) induced from iPS cells as described above. Analysis results from the flow cytometer of immature T cells by cell surface antigen markers are shown in FIG. 7. In the immature T cells induced from IPS cells, expression of CD45+ TCR αβ- CD3+ CD4± CD8 αβ+ which is expressed *in vivo* in mature cytotoxic T cells was confirmed.

### Example 3

### Production of iPS cell clones

Mononuclear cells were isolated from peripheral blood collected from patients with hepatoma or hepatoblastoma using mononuclear cell separation solution Lymphoprep^{®}. From the obtained mononuclear cells, CD19/CD20 positive B cells and CD3/CD4/CD8 positive T cells were removed using FACS or MACS beads to obtain non-T non-B cells or monocytes. The obtained non-T non-B cells or monocytes population were infected with Sendai virus (CytoTune^{®} 2.0) loaded with Yamanaka's four factors (Oct3/4, Sox2, Klf4, and c-Myc) and Sendai virus that had coded for SV40Tag, at an MOI (multiplicity of infection) of 5 to 20. It should be noted that the SV40 may be removed.

The obtained iPS cells consist of a number of iPS cell clones. For this reason, colonies were picked up and cloned. All cloned iPS cells were cryopreserved. The cloned iPS cells were cultured in a differentiation medium for about 10 days to induce hematopoietic stem cells, and CD34/CD43 double-positive hematopoietic stem cells were isolated. The isolated hematopoietic stem cells were cultured for about 21 days on a plate coated with FcDLL4, a fusion protein of DLL4 protein and Fc region of immunoglobulin, then differentiated and induced into T cells.

The frequency of obtaining immature cytotoxic T cells after the culture for 21 days was verified by the CD8 α chain/β chain double-positive rate, and the clones with the highest appearance frequency of the CD8 α chain/β chain double-positive cells were selected.

A master cell bank was constructed by expanded culture of several iPS cell clones without good differentiation efficiency into T cells in an iPS cell maintenance medium for two weeks, then dispensing into a cell storage container, followed by cryopreservation.

### Example 4

### Transfection of TCR genes to iPS cell clones

To the iPS cell clones which was obtained in Example 2 and derived from non-T non-B cells or monocytes with good differentiation efficiency into T cells, a piggyBac vector having the genes encoding a TCR α chain and a β chain in which tumor antigen specificity had been confirmed was transfected using electroporation method. Then, the iPS cells expressing the targeted TCR α and β chains were isolated by a cell sorter using the expression of CD19, a marker molecule, as an indicator. The isolated iPS cells were cultured for about 10 days in a differentiation medium and CD34/CD43 double-positive hematopoietic stem cells were induced and isolated by a cell sorter. The isolated blood stem cells were cultured for about 21 days on a plate coated with FcDLL4, and differentiated and induced into T cells.

The CD8 α-chain / β-chain double-positive immature T cells obtained after the culture for 21 days were isolated and purified using a cell sorter. Then, the immature T cells were co-cultured in the presence of PHA (phytohemagglutinin) and peripheral blood mononuclear cells as feeder cells, in the presence of RetroNectin^{®} and anti-CD3 antibodies, or in the presence of anti-CD3 antibodies and anti-CD28 antibodies to induce mature cytotoxic T cells. These stimulations were carried out once or more. The performance of the obtained T cells was confirmed by the cytotoxic activity of the GPC3-specific target cells, IFN-γ production, and antigen binding ability.

### Example 5

### Transfection of TCR genes to mature T cells differentiated from iPS cell clones

To mature T cells differentiated from the iPS cell clones which were obtained in Example 3 and derived from non-T non-B cells or monocytes with good differentiation efficiency into T cells via hematopoietic stem cells and immature T cells, genes (cDNAs) encoding a GPC3 antigen-specific TCR α chain and β chain were transfected using a piggyBac^{®} system in the same way as that in Example 4.

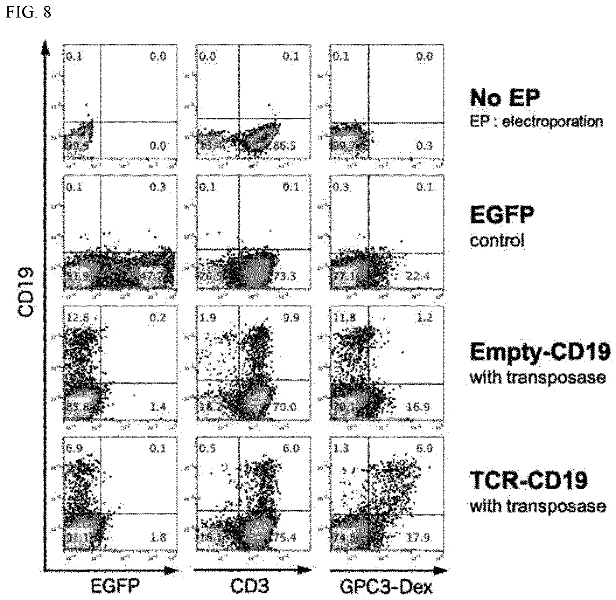

The results of analyzing the phenotype of the mature T cells derived from the transfected iPS cells with a flow cytometer are shown in FIG. 8. In FIG. 8, "No EP" shows the analytical results of the mature T cells used in transfection, and in which the WT1 antigen-specific TCR α chain and β chain are expressed. "EGFP" shows the analytical results of the mature T cells transfected with an expression vector incorporating a tracer gene (EGFP (enhanced green fluorescent protein) gene) as an indicator of transfection manipulation. "Empty-CD19" shows the analytical results of the mature T cells transfected with a PiggyBac^{®} transposon vector incorporating only an intracellularly defective human CD19 gene as a tracer gene and a transposon vector. "TCR-CD19" shows the analytical results of the mature T cells transfected with a PiggyBac^{®} transposon vector incorporating a GPC3 antigen-specific TCR α-chain β-chain gene and an intracellularly defective human CD19 gene in tandem.

In the iPS cell-derived mature T cells represented by "No EP", only CD3, a T cell marker, was detected. That is, the cells used for transfection were confirmed to be T cells.

In the iPS cell-derived mature T cells represented by "EGFP", expression of CD3 genes and EGFP genes was detected. That is, it can be seen that the transfection manipulation had been appropriately performed.

In the iPS cell-derived mature T cells represented by "Empty-CD19", the expression of CD3 gene and intracellular defective human CD19 gene, which is a tracer gene incorporated into piggyBac^{®} transposon vector was detected. That is, it can be seen that the transfection manipulation had been appropriately performed.

In the iPS cell-derived mature T cells represented by "TCR-CD19", in addition to expression of CD3 gene and intracellular defective human CD19 gene, which is a tracer gene incorporated into piggyBac^{®} transposon vector, the binding to GPC3 peptide/HLA complex (GPC3-Dex) recognized by GPC3 antigen-specific TCR α chain and β chain was detected. That is, it has been shown that by transfecting a GPC3 antigen-specific TCR α chain and β chain gene into iPS cell-derived mature T cells using a piggyBac^{®} system, a GPC3 antigen-specific TCR α chain and β chain expressed on the cells function as molecules that recognize a GPC3 peptide/HLA complex (GPC3-Dex). Therefore, it has been shown that by using novel tumor-related antigens (neoantigens) and TCR α chain and β chain genes which are specific to other tumor-related antigens as TCR α chain and β chain genes to be transfected into iPS cell-derived mature T cells, iPS cell-derived mature T cells that recognize these antigens can be produced.

### Example 6

### Production of regenerated T cells from iPS cells in which peripheral blood T cells have been reprogramed

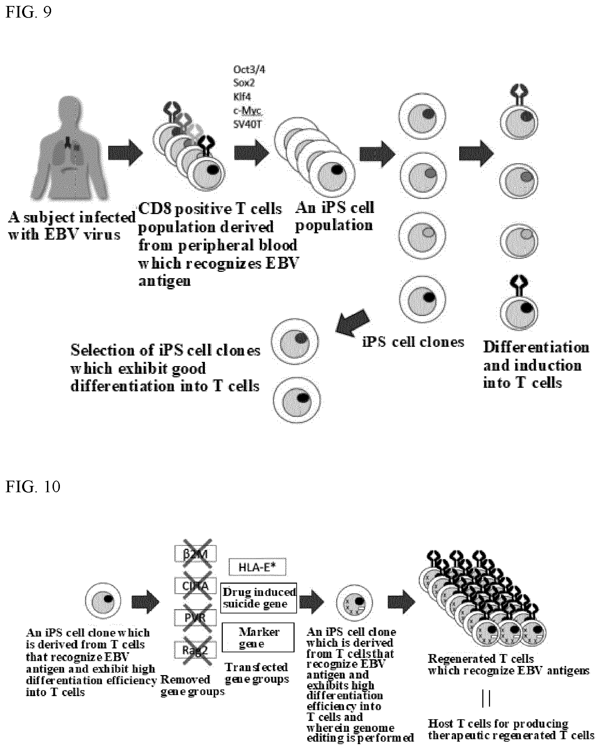

FIG. 9 shows a method for selecting the iPS cell clones that demonstrate high differentiation efficiency into T cells in the step of producing regenerated T cells from iPS cells in which peripheral blood T cells have been reprogramed. From the peripheral blood of a subject infected with EBV (Epstein-Barr virus), mononuclear cells were separated. The separated mononuclear cells were stimulated with EBV antigen in a test tube. The CD8-positive T cell populations that recognize the EBV antigen were isolated. To the isolated CD8-positive T cell populations, Yamanaka's four factors (Oct3/4, Sox2, Klf4, and c-Myc) and SV40T antigen were introduced using a Sendai virus vector to obtain iPS cell populations. From the obtained iPS cell populations, iPS cell clones were fractionated. For each clone, the differentiation potential to T cells was examined, and the iPS cell clones that demonstrate high differentiation efficiency into T cells were selected. T cells that recognize EBV antigens are the cells that are less likely to cause alloreaction even in a case of allografting.

FIG. 10 shows a method for performing genome editing on iPS cell clones selected as the cells with high differentiation efficiency into T cells and producing regenerated T cells in the step of producing regenerated T cells from iPS cells in which peripheral blood T cells have been reprogramed. Regarding the iPS cell clones which are derived from CD8-positive T cells that recognize EBV antigen and demonstrate high differentiation efficiency into T cells, the β2M and CIITA genes involved in the expression of MHC class I and MHC class II, PVR genes involved in activation of natural killer (NK) cells, and Rag2 gene involved in rearrangement of T-cell receptor were deleted using CRISPR/Cas9. On the other hand, a fusion gene (HLA-E*) of β2M/binding peptide/HLA-E, which is an inhibitory ligand for NK cells, was expressed to make iPS cells immune to attack from T cells and NK cells of host. In this genome editing, cells can be removed after administration into a living body by expressing a suicide gene such as a drug-induced caspase 9 and/or a specific marker gene (EGFR (epidermal growth factor receptor), CD19, and CD20). After the genome editing, re-cloning was carried out. When the high differentiation efficiency to T cells was confirmed, the iPS cell-derived regenerated T cells may be preserved as clones to construct a master cell bank. The regenerated T cells differentiated from iPS cells and proliferated are host T cells for producing regenerated T cells for cancer treatment, and a master cell bank may be constructed by the host T cells. The host T cells can be used as a material for producing regenerated T cells into which TCR gene or CAR (chimeric antigen receptor) gene has been transfected. Since the host T cells are the T cells that recognize EBV antigen, they are unlikely to cause an alloreaction even when being transferred into a living body. "Host T cells" mean the T cells used as a starting material for producing a preventive or therapeutic agent for cancer containing regenerated T cells as an active ingredient, although the cells themselves are not subjected to treatment in patients.

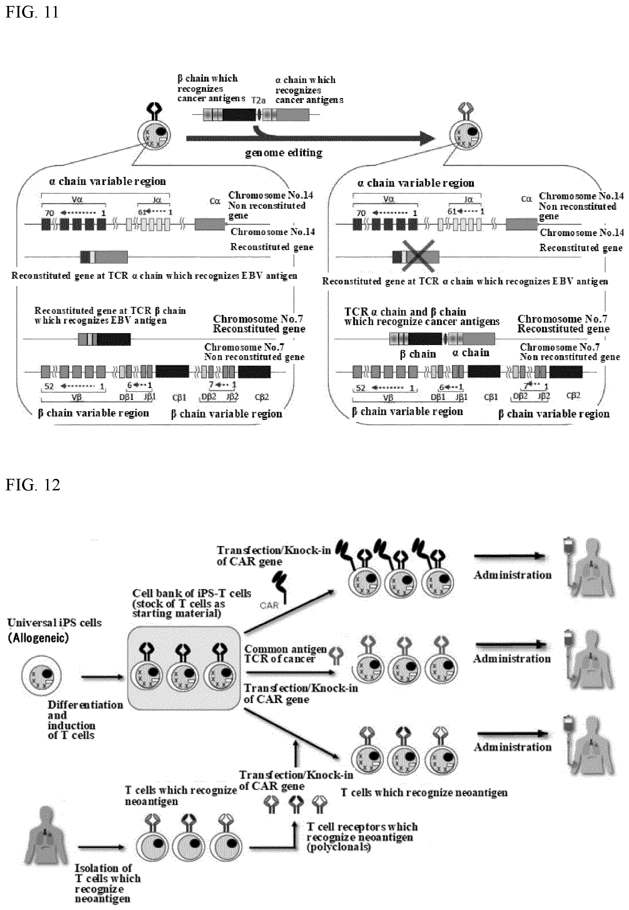

FIG. 11 shows a method for producing regenerated T cells which recognize cancer antigens from the host T cells. In the host T cells which recognize EBV antigens, the TCR β chain which recognize rearranged EBV antigen was replaced with a T2a-mediated conjugation of the TCR β chain and the TCR α chain, which recognize cancer antigens, respectively, by gene replacement according to genome editing using CRISPR/Cas9. On the other hand, the TCR α chain which recognizes EBV antigen was removed by genome editing using CRISPR/Cas9. According to the method described in FIG. 11, it was possible to produce regenerated T cells that recognize cancer antigens.

FIG. 12 summarizes the methods shown in FIGs. 9 to 11. The regenerated T cells produced from the same type of universal iPS cells in which peripheral blood T cells have been reprogramed (iPS-T cells) can be used as starting materials for producing T cells transfected with TCR genes or CAR genes. "Universal iPS cells" mean the iPS cells that can be used without causing a rejection even to a patient having any kind of MHC (major histocompatibility complex) due to low immunogenicity. That is, they are the iPS cells which can be administered to a patient without considering MHC matching. Universal iPS cells can be produced by knocking out MHC class I or MHC class II molecules and expressing ligands that inhibit NK cells.

For the transfection of TCR genes or CAR genes, viral vectors used in the production of T cells for conventional replenishment therapy or regenerative therapy with T cells may be used. By using iPS-T cells as a starting material, it becomes possible to produce desired T cells as an active ingredient of preventive or therapeutic agent for cancer in a short period of time. In addition, it is also easy to transfect the TCR that recognizes neoantigens, and it is also possible to produce iPS-T cells while maintaining multi-clone properties by transfecting a plurality of TCR genes that recognize different neoantigens.

## Claims

1. A cell bank composed of cells for transfecting a T cell receptor gene, wherein the cells are one or more kinds of cells selected from the group consisting of iPS cells, hematopoietic stem cells differentiated from the iPS cells, immature T cells, and mature T cells.

2. The cell bank according to claim 1, wherein the cells are those for further transfecting a chimeric antigen receptor gene.

3. The cell bank according to claim 1 or 2, wherein the iPS cells are peripheral blood mononuclear cells of a subject and are those obtained by reprogramming the peripheral blood mononuclear cells from which B cells and T cells have been removed.

4. The cell bank according to claim 3, wherein the transfection of T cell receptor gene into the iPS cell clone or the hematopoietic stem cells differentiated from the iPS cell clone or immature T cells uses a viral vector, a transposon vector, or genome editing technique.

5. The cell bank according to claim 1 or 2, wherein the iPS cells are those obtained by reprogramming T cells of a subject.

6. The cell bank according to claim 5, wherein the transfection of T cell receptor gene into the mature T cells uses genome editing technique.

7. The cell bank according to any one of claims 1 to 6, wherein the iPS cells are iPS cell clones with good differentiation efficiency into mature T cells.

8. The cell bank according to any one of claims 1 to 7, wherein the cells are cryopreserved.

9. The cell bank according to claim 5, wherein the cells are those genetically modified so that expression of endogenous T cell receptors can be controlled.

10. The cell bank according to any one of claims 1 to 9, wherein the hematopoietic stem cells and the immature T cells are the cells which do not express a T cell receptor.

11. The cell bank according to claim 5, wherein the mature T cells express a T cell receptor which does not recognize non-tumor cells derived from a subject different from a subject from which the mature T cells are derived.

12. The cell bank according to claim 11, wherein the mature T cells recognize a single antigen.

13. The cell bank according to claim 12, wherein the single antigen is influenza virus antigen, EB virus antigen, HPV antigen, HBV antigen, HCV antigen, HIV antigen, coronavirus antigen, or HTLV antigen.

14. The cell bank according to any one of claims 1 to 13, wherein the hematopoietic stem cells are CD34/CD43 double-positive.

15. The cell bank according to any one of claims 1 to 14, wherein the immature T cells are CD8 α chain/β chain double-positive.

16. The cell bank according to any one of claims 1 to 15, wherein the mature T cells are CD8 α chain/β chain double-positive and TCR α chain/β chain double-positive.

17. The cell bank according to any one of claims 1 to 16, wherein the T cell receptor gene is prepared for each single cell from a T cell population which is T cells obtained from a subject and has reactivity to tumor-related antigen.

18. The cell bank according to any one of claims 1 to 16, wherein the T cell receptor gene is prepared for each single cell from the T cell population which has reactivity to tumor-related antigen by contacting the T cells obtained from a subject with a tumor-related antigen.

19. The cell bank according to any one of claims 1 to 16, wherein the T cell receptor gene is prepared for each single cell from the T cell population which has reactivity to tumor-related antigen by contacting the T cell obtained from a subject to which a tumor-related antigen has been administered with the tumor-related antigen.

20. The cell bank according to any one of claims 17 to 19, wherein the tumor-related antigen is selected from the group consisting of GPC3, WT1, XAGE1, LMP2, NY-ESO-1, EB virus antigen and neoantigen, as well as peptide fragments thereof.

21. The cell bank according to any one of claims 17 to 19, wherein the tumor-related antigen is EYILSLEEL (SEQ ID NO: 1) which is an HLA-A24-binding GPC3 peptide, FVGEFFTDV (SEQ ID NO: 2) which is an HLA-A2-binding GPC3 peptide, or a mixture thereof.

22. The cell bank according to any one of claims 17 to 19, wherein the T cell population is CD3/CD137 double-positive.

23. The cell bank according to any one of claims 17 to 19, wherein the T cell population binds to MHC tetramer or MHC Dextran^{®} which forms a complex with the tumor-related antigen peptide.

24. The cell bank according to any one of claims 1 to 23, wherein the subject providing cells for obtaining the iPS cells and the subject providing cells for preparing the T cell receptor gene are the same individual.

25. The cell bank according to any one of claims 1 to 23, wherein the subject providing cells for obtaining the iPS cells and the subject providing cells for preparing the T cell receptor gene are separate individuals from each other.

26. The cell bank according to any one of claims 1 to 25, wherein the cells for transfecting a T cell receptor gene or a T cell receptor gene and a chimeric antigen receptor are intermediates for producing T cell formulations used in prevention and/or treatment of cancer.

27. Use of the cell bank according to any one of claims 1 to 26 for production of T cell formulations used in prevention and/or treatment of cancer.

28. Regenerated T cells produced from the cell bank according to any one of claims 1 to 26.

29. A pharmaceutical composition containing the regenerated T cells according to claim 28.

30. A method for preventing or treating cancer using the pharmaceutical composition according to claim 29.
